# EUROPÄISCHE PATENTSCHRIFT

(11) **EP 2 173 702 B1**
(45) Veröffentlichungstag und Bekanntmachung des Hinweises auf die Patenterteilung: **04.07.2018**
(21) Anmeldenummer: 06791291.5
(22) Anmeldetag: 10.05.2006
(51) Int. Cl.: C07C 201/12, C07C 253/30, C07C 255/64, C07C 41/30, C07C 25/18, C07C 315/04, C07C 317/14, C07C 209/68, C07C 49/78, C07C 25/24, C07C 49/84, C07C 45/72, C07C 45/68, C07C 211/55, C07C 233/07, C07C 319/20, C07C 205/57, C07C 43/205, C07B 37/04, B01J 31/26

(54) **VERFAHREN ZUR DECARBOXYLIERENDEN C-C VERKNÜPFUNG VON CARBONSÄUREN MIT KOHLENSTOFFELEKTROPHILEN**
METHOD FOR DECARBOXYLATING C-C CROSS-LINKING OF CARBOXYLIC ACIDS WITH CARBON ELECTROPHILES
PROCÉDÉ DE COUPLAGE C-C DE DÉCARBOXYLATION D'ACIDES CARBOXYLIQUES À L'AIDE DES ÉLECTROPHILES DE CARBONE

(30) Priorität: 10.05.2005 DE 102005022362
(43) Veröffentlichungstag der Anmeldung: 14.04.2010
(73) Patentinhaber: Studiengesellschaft Kohle mbH, 45470 Mülheim an der Ruhr (DE)
(72) Erfinder: GOOSSEN, Lukas, 67663 Kaiserslautern (DE); DENG, Guo-Jun,, 52074 Aachen (DE)
(86) Internationale Anmeldenummer: PCT/DE2006/001014
(87) Internationale Veröffentlichungsnummer: WO 2006/136135

(56) Entgegenhaltungen:
- EP-A1- 0 606 065
- WO-A-97/33846
- M. NILSSON: "A NEW BIARYL SYNTHESIS ILLUSTRATING A CONNECTION BETWEEN THE ULLMANN BIARYL SYNTHESIS AND COPPER-CATALYSED DECARBOXYLATION" ACTA CHEM. SCAND., Bd. 20, Nr. 2, 1966, Seiten 423-426, XP009075993
- IIHAMA T ET AL: "REGIOSPECIFIC SYNTHESES OF ALL ISOMERIC NITROFLUORENONES AND NITROFLUORENES BY TRANSITION METAL CATALYZED CROSS-COUPLING REACTIONS" SYNTHESIS, GEORG THIEME VERLAG, STUTTGART, DE, Nr. 3, 1. März 1989 (1989-03-01), Seiten 184-187, XP000022712 ISSN: 0039-7881
- LUKAS J. GOOSSEN ET AL: "Low-Temperature Ag/Pd-Catalyzed Decarboxylative Cross-Coupling of Aryl Triflates with Aromatic Carboxylate Salts", CHEMISTRY - A EUROPEAN JOURNAL, vol. 16, no. 13, 6 April 2010 (2010-04-06) , pages 3906-3909, XP55194402, ISSN: 0947-6539, DOI: 10.1002/chem.200903319
- Nuria Rodriguez, Lukas J. Goossen: "Decarboxylative coupling reactions: a modern strategy for C-C-bond formation", Chem. Soc. Rev, 1 January 2011 (2011-01-01), pages 5030-5048, XP055338610, DOI: 10.1039/C1CS15093F Retrieved from the Internet: URL:http://pubs.rsc.org/en/content/article pdf/2011/cs/c1cs15093f [retrieved on 2017-01-25]

## Beschreibung

Die Erfindung betrifft ein Verfahren zur C-C Verknüpfung von Kohlenstoffnucleophilen, die durch Decarboxylierung aus Carbonsäuresalzen gebildet werden, mit Kohlenstoffelektrophilen in Gegenwart von Übergangsmetallkatalysatoren.

Die Biaryl-Substruktur ist eine Schlüsselfunktionalität in Funktionsmaterialien, Pharmaka und Pflanzenschutzpräparaten. Zur Synthese solcher Verbindungen wird insbesondere die Suzuki-Kupplung von Arylhalogeniden mit Boronsäuren genutzt, da sie einfach und in hervorragenden Ausbeuten durchführbar ist, und viele funktionelle Gruppen und Substitutionsmuster toleriert werden. Von besonderem Interesse ist die Verbindung 2-Nitro-4'chlorbiphenyl, eine Zwischenstufe bei der Darstellung des Fungizids Boscalid. Ihre industrielle Darstellung ist als Beispiel für eine Suzuki-Kupplung in Schema 1 dargestellt.

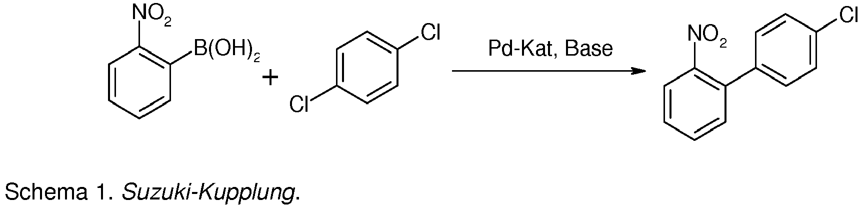

Insbesondere für industrielle Anwendungen hat diese Vorgehensweise jedoch aufgrund der schwierigen Zugänglichkeit und dem daraus resultierenden hohen Preis von Boronsäuren gravierende Nachteile. Ihre Synthese durch Metallierung von Vinyl- und Arylhalogeniden und nachfolgende Umsetzung mit Trialkylboraten ist aufwendig und nicht mit allen funktionellen Gruppen verträglich. Breiter einsetzbar sind katalytische Kreuzkupplungsreaktionen von Organohalogeniden mit Diborverbindungen, aber aufgrund des hohen Preises dieser Verbindung finden sie kaum technische Verwendung. Auch die Darstellung von Boronsäuren aus Pinacol- bzw. Catecholboran ist aufgrund der hohen Kosten industriell wenig attraktiv.

Andere Biarylsynthesen sind der Suzuki-Kupplung hinsichtlich Ausbeute, Durchführbarkeit und Anwendungsbreite in der Regel deutlich unterlegen. Beispielsweise liefert die Scholl-Reaktion, bei der zwei Arene in Gegenwart einer Lewis- und einer Brönstedt-Säure miteinander umgesetzt werden, in der Regel nur unbefriedigende Ausbeuten und ist nur mit wenigen funktionellen Gruppen verträglich. Ähnliches gilt für die Gomberg-Bachmann-Reaktion. Breiter einsetzbar ist die Ullmann-Reaktion, bei der zwei Aryliodide in Gegenwart von Kupferverbindungen miteinander gekuppelt werden, jedoch gelingt nur die Synthese symmetrischer Verbindung in befriedigender Ausbeute.

Katalytische Kreuzkupplungsreaktionen mit Mg-, Zn- oder Li-Arylen sind aufgrund der schwierigen Handhabung dieser Verbindungen und ihrer geringen Verträglichkeit mit funktionellen Gruppen weniger vorteilhaft als die Suzuki-Reaktion. Lediglich Arylsiloxane und die toxischen Arylstannane zeigen ähnlich gute Eigenschaften wie Arylboronsäuren, allerdings ergeben sich bei der Darstellung dieser Verbindungen auch die gleichen Probleme.

Es bestand somit Bedarf an einer neuen Kreuzkupplungsreaktion ausgehend von Kohlenstoffnucleophilen, die sich durch gute Verfügbarkeit und Handhabbarkeit sowie einen niedrigen Preis auszeichnen. Metallsalze von Carbonsäuren sind vielfältig verfügbar und stellen daher ideale Startmaterialien dar. Eine Kupplungsreaktion, bei der Organometallspezies durch Decarboxylierung von Carbonsäuresalzen *in situ* erzeugt und mit Kohlenstoffelektrophilen gekuppelt werden, ist daher von hohem Interesse, insbesondere, wenn sie sich auch zur Erzeugung von Biarylen nutzen lässt.

Bisher gibt es nur ein Beispiel (Nilsson, ACTA CHEM. SCAND., Bd. 20, Nr. 2, 1966, Seiten 423-426) für eine artverwandte Reaktion, in welcher sich bei der Pyrolyse von Kupfercarboxylaten in Gegenwart von überschüssigem Arylhalogenid als Bestandteil eines komplexen Reaktionsgemisches unter anderem auch das entsprechende Biaryl bildet. Die extremen Temperaturen (240°C), die niedrigen Ausbeuten, die großen Mengen an Kupfersalzen und die beschränkte Substratbreite machen diese Reaktion jedoch für eine kommerzielle Nutzung ungeeignet.

Die Aufgabe der vorliegenden Erfindung war es, ein allgemein anwendbares, katalytisches Verfahren zur Kreuzkupplung von Kohlenstoffnucleophilen mit Kohlenstoffelektrophilen zu entwickeln, in dem die Kohlenstoffnucleophile *in situ* durch Decarboxylierung von Metallcarboxylaten gebildet werden. Die besondere Schwierigkeit bestand in der Auffindung eines geeigneten Katalysatorsystems, da eine Decarboxylierung von Carbonsäuren üblicherweise über einen radikalischen Mechanismus erfolgt, während die Effizienz von Kreuzkupplungskatalysatoren darin begründet ist, dass sie selektiv nur Zweielektronenprozesse fördern und radikalische Reaktionsschritte zurückdrängen.

Gegenstand der vorliegenden Erfindung ist Verfahren zur decarboxylierenden C-C Verknüpfung durch Umsetzung von Carbonsäuresalzen mit Kohlenstoffelektrophilen in Gegenwart eines Katalysatorsystems, der zwei oder mehr Metallverbindungen enthält.

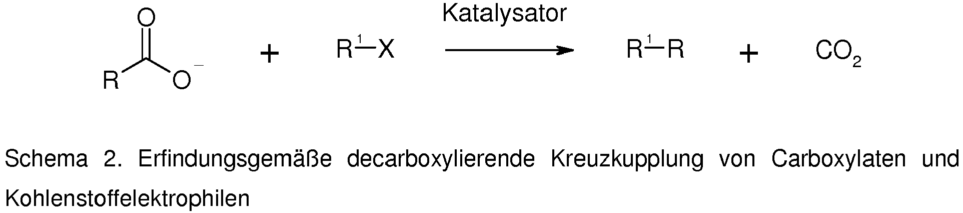

Überraschenderweise wurde gefunden, dass ein Katalysatorsystem, dass zwei oder mehr Metallverbindungen enthält, die decarboxylierende Kreuzkupplung von Carbonsäuresalzen mit Kohlenstoffelektrophilen gemäß Schema 2 sehr effizient katalysieren kann.

Erfindungsgemäß enthält das Katalysatorsystem zwei verschiedene Übergangsmetallverbindungen. Das sind solche Systeme, in denen das erste Übergangsmetall Oxidationsstufen annehmen kann, die sich um eine Einheit unterscheiden, und das zweite Oxidationsstufen annehmen kann, die sich um zwei Einheiten voneinander unterscheiden. Es wird angenommen, dass das erste Übergangsmetall eine radikalische Decarboxylierung katalysiert, während das zweite die Zweielektronenprozesse einer Kreuzkupplungsreaktion katalysiert. Überaschenderweise stören sich diese simultan ablaufenden katalytischen Prozesse nicht, es wird vermutet, dass die Katalysatorkomponenten sich in ihrer Wirkung gegenseitig verstärken, so dass der Decarboxylierungsschritt auch bei extrem niedrigen Katalysatormengen schon bei beispiellos niedrigen Temperaturen erfolgt.

Sofern zwei oder mehr unterschiedliche Übergangsmetalle oder -metallverbindungen eingesetzt werden, können in Abhängigkeit vom eingesetzten Substrat das erste Metallverbindung als erste
Katalysatorkomponente in stöchiometrischen Mengen und das zweite Metall bzw. Metallverbindung als zweite Katalysatorkomponente in katalytischen Mengen eingesetzt werden.

Weiterhin wurde ein Verfahren gefunden, in dem dieses Katalysatorsystem zum Einsatz kommt, das dadurch gekennzeichnet ist, dass Carbonsäuresalze unter Extrusion von Kohlendioxid in Kohlenstoffnucleophile überführt werden und mit Kohlenstoffelektrophilen unter Ausbildung einer C-C-Bindung verknüpft werden.

Vorteilhaft gegenüber dem beschriebenen Stand der Technik ist, dass Carbonsäuresalze wesentlich preisgünstiger, einfacher verfügbar und besser handhabbar sind als metallorganische Verbindungen, beispielsweise Boronsäuren. Die Vorteile des neuen Katalysators gegenüber dem von Nilsson beschriebenen ergeben sich daraus, dass zur Bildung des erfindungsgemäßen Katalysatorsystems eine zweite Metallkomponente zugesetzt wird, die speziell die Kreuzkupplung katalysiert. Auf diese Weise kann eine weitaus größere Substratbreite bei weitaus niedrigeren Temperaturen in beispiellos hohen Selektivitäten und Ausbeuten umgesetzt werden. Zudem werden nur katalytische Mengen an Übergangsmetallen benötigt, während das Verfahren von Nilsson unbedingt stöchiometrische Mengen and Kupfer erfordert.

Das erfindungsgemäße Katalyatorsystem enthält zwei Metallkomponenten. Die erste Komponente ist dadurch gekennzeichnet, dass sie ein Metall enthält, das zwei Oxidationsstufen einnehmen kann, die sich um eine Einheit unterscheiden, nämlich Cu (0,I,II) (in Klammern beispielhaft geeignete Kombinationen von Oxidationstufen). Das Metall kann wahlweise in elementarer Form, als Komplex oder als Salz eingesetzt werden. Besonders bevorzugt werden Kupfer(I)verbindungen eingesetzt.

Die zweite Komponente ist dadurch gekennzeichnet, dass sie ein Metall enthält, das zwei Oxidationsstufen einnehmen kann, die sich um zwei Einheiten unterscheiden, nämlich Pd (0,II) (in Klammern beispielhaft geeignete Kombinationen von Oxidationstufen). Das Metall kann wahlweise in elementarer Form, als Komplex oder als Salz eingesetzt werden. Besonders bevorzugt wird Palladium(II)acetylacetonat eingesetzt.

Von beiden Metalle ist zumindest eines durch weitere Liganden aus der Reihe Amine, Phosphine, N-heterocyclische Carbene, Nitrile oder Olefine stabilisiert. Besonders bevorzugt werden als Liganden cyclische Amine verwendet, ganz besonders bevorzugt werden chelatisierende cyclische Amine aus der Reihe Phenanthrolin, Bipyridin und Terpyridin oder ihre substituierten Derivate verwendet.

Wahlweise können die eigentlichen Katalysatoren aus geeigneten Metallvorstufen durch den Zusatz der oben aufgeführten Komponenten im Reaktionsgemisch erzeugt werden.

Im erfindungsgemäßen Verfahren werden Carbonsäuresalze der allgemeinen Formel I eingesetzt, wobei n, m, q, p Zahlen zwischen 1 und 6 sind und r eine Zahl zwischen 0 und 6 ist.

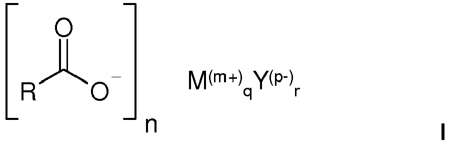

Das Gegenion M⁽ⁿ⁺⁾ ist dabei wahlweise ein Metallkation, bevorzugt aus der Reihe der Metalle Li, Na, K, Mg, Ca, B, Al, Ag, Cu, Mn, Fe, Co, Ni, Mo, Ru, oder ein organisches Kation, bevorzugt aus der Reihe Ammonium, Pyridinium, Phosphonium. Wahlweise kann zusätzlich zum Carboxylat noch ein weiteres Anion koordiniert sein, vorzugsweise aus der Reihe I⁻, Br⁻, Cl⁻, F⁻, CO₃⁻⁻, CN⁻, OH⁻, OAlkyl⁻, HCO₃⁻,PO₄³⁻, HPO₄ ²⁻, H₂PO4⁻.

Die Carbonsäuresalze werden wahlweise in präformierter Form zugesetzt oder *in situ* aus den Carbonsäuren und geeigneten Basen erzeugt.

Der Substituent R ist ein organischer Rest, wählbar aus der Reihe lineare, verzweigte und zyklische C₁ - C₂₀-Alkyl, lineare, verzweigte und zyklische C₂ - C₂₀- Alkenyl- sowie C₆- C₂₀-Aryl und Heteroaryl aus der Reihe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, und kann seinerseits weitere Substituenten aus der Reihe lineare, verzweigte und zyklische C₁ - C₁₀-Alkyl, lineare, verzweigte und zyklische C₂ - C₂₀- Alkenyl- sowie C₆-C₁₀-Aryl und Heteroaryl, lineare, verzweigte und zyklische C₁-C₁₀-Alkyloxy oder C₁-C₁₀-Aryloxy, lineare, verzweigte und zyklische C₁ - C₁₀ Alkyl- oder C₁ - C₁₀-Arylaminocarbonyl, lineare, verzweigte und zyklische C₁ - C₁₀ Acyl, lineare, verzweigte und zyklische C₁ - C₁₀ Dialkylamino, C₁ - C₁₀ Arylamino, Formyl, Oxo, Thio, Hydroxy, Carboxyl, Nitro, Cyano, Nitroso, und Halogene wie F, Cl, Br und I tragen.

Bevorzugt ist R wählbar aus der Reihe lineare, verzweigte und zyklische C₂ - C₁₀- Alkenyl-, C₆- C₁₀-Aryl oder Heteroaryl aus der Reihe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, und kann seinerseits weitere Substituenten tragen, wie im vorigen Absatz beschrieben.

R1-X (II)

Beim Kohlenstoffelektrophil R¹-X (Formel II) ist der Substituent X eine gängige Abgangsgruppe, wählbar aus der Reihe: Halogenide sowie Pseudohalogenide aus der Reihe Toluolsulfonat, Methylsulfonat, Trifluorsulfonat, Trifluoracetat, Carboxylat und [N₂]⁺.

Gemäß dem erfindungsgemäßen Verfahren ist der Substituent R¹ des Kohlenstoffelektrophils (Formel II) ein organischer Rest, wählbar aus der Reihe lineare, verzweigte und zyklische C₁ - C₂₀-Alkyl, lineare, verzweigte und zyklische C₂ - C₂₀- Alkenyl- sowie C₆- C₂₀-Aryl und Heteroaryl aus der Reihe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, und kann seinerseits weitere Substituenten aus der Reihe lineare, verzweigte und zyklische C₁ - C₁₀-Alkyl, lineare, verzweigte und zyklische C₂ - C₂₀- Alkenyl- sowie C₆-C₁₀-Aryl und Heteroaryl, lineare, verzweigte und zyklische C₁-C₁₀-Alkyloxy oder C₁-C₁₀-Aryloxy, lineare, verzweigte und zyklische C₁ - C₁₀ Alkyl- oder C₁ - C₁₀-Arylaminocarbonyl, lineare, verzweigte und zyklische C₁ - C₁₀ Acyl, lineare, verzweigte und zyklische C₁ - C₁₀ Dialkylamino, C₁ - C₁₀ Arylamino, Formyl, Oxo, Thio, Hydroxy, Carboxyl, Nitro, Cyano, Nitroso, und Halogene wie F, Cl, Br und I tragen.

Bevorzugt ist R¹ wählbar aus der Reihe lineare, verzweigte und zyklische C₂ - C₁₀- Alkenyl-, C₆- C₁₀-Aryl oder Heteroaryl aus der Reihe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, und kann seinerseits weitere Substituenten tragen, wie im vorigen Absatz beschrieben.

Alternativ kann es sich bei dem Kohlenstoffelektrophil um ein Carbonylderivat handeln, aus der Reihe Carbonsäurechlorid, Carbonsäureanhydrid, Aldehyd, Keton, Ester, α,β-ungesättigtes Aldehyd, α,β-ungesättigtes Keton, α,β-ungesättigter Ester.

Gemäß dem erfindungsgemäßen Verfahren werden beiden Katalysatoren unabhängig voneinander in Mengen von 0,001 mol% bis 100 mol% bezogen auf das Kohlenstoffelektrophil eingesetzt, bevorzugt werden Mengen von 0,001 mol% bis 10 mol% eingesetzt und besonders bevorzugt Mengen von 0,01 mol% bis 5 mol%.

Das erfindungsgemäße Verfahren wird bei Temperaturen von 20 °C bis 220 °C, vorzugsweise bei 80 °C bis 200 °C und besonders bevorzugt bei 120 °C bis 160 °C durchgeführt.

Das erfindungsgemäße Verfahren kann in Gegenwart eines Lösungsmittels oder in Substanz durchgeführt werden. Beispielsweise können als Lösungsmittel einer der Einsatzstoffe, lineare, zyklische und verzweigte Kohlenwasserstoffe (beispielsweise Hexane, Heptane und Octane), aromatische Kohlenwasserstoffe (beispielsweise Benzol, Toluol, Xylole, Ethylbenzol, Mesitylen), Ether (beispielsweise 1,4-Dioxan, Tetrahydrofuran, Methyltetrahydrofuran, Dibutylether, Methyl-t-butylether, Diisopropylether, Diethylenglycoldimethylether), Ester (beispielsweise Ethylacetat, Butylacetat), Amide (beispielsweise Dimethylformamid, Diethylformamid, N-Methylpyrrolidon, Dimethylacetamid), Dimethylsulfoxid, Sulfolan, Acetonitril, Isobutyronitril, Propionitril, Propylencarbonat und chlorierte aliphatische und aromatische Kohlenwasserstoffe eingesetzt werden.

Bevorzugt werden Dimethylformamid, Diethylformamid, N-Methylpyrrolidon, Dimethylacetamid, Dimethylsulfoxid, Sulfolan, Acetonitril und Propylencarbonat eingesetzt.

Das erfindungsgemäße Verfahren wird vorzugsweise so durchgeführt, dass Spuren von Wasser während der Reaktion durch übliche Methoden entfernt werden, beispielsweise durch Destillation oder durch Zusatz von Wasser bindenden Mitteln.

Ein weiterer Gegenstand der vorliegenden Erfindung ist ein Katalysatorsystem zur decarboxylierenden C-C Verknüpfung von Carbonsäuresalzen mit Kohlenstoffelektrophilen, bestehend aus einer Palladiumverbindung und einer Kupferverbindung und einem wahlweise substituierten, chelatisierenden cyclischen Amin aus der Reihe Phenanthrolin, Bipyridin und Terpyridin.

### Beispiele 1-18

Aus den Beispielen 1-18 geht hervor, dass mit einer Kombination aus Kupfer(II)carbonat und Palladium-Phosphinkomplexen bei besonders niedrigen Temperaturen von 120° exzellente Ausbeuten an Kreuzkupplungsprodukten erzielt werden. Die eingesetzten Übergangsmetallverbindungen werden im Reaktionsgemisch zu niedrigeren Oxidationsstufen reduziert. Es wird angenommen, dass Kupfer als Cu(I)-Verbindungen und Palladium als Pd(O)
vorliegt.

Dabei wurde gemäß Tabelle 1 jeweils zunächst 1,5 mmol o-Nitrobenzoesäure mit einer stöchiometrischen Menge der Base deprotoniert. Das resultierende Carbonsäuresalz wurde mit 1,0 mmol 4-Chlor-1-brombenzol in Gegenwart eines Palladium(II)salzes (0,02 mmol), gegebenenfalls einem Liganden (0,06 mmol) und/oder einem Additiv (1,5 mmol) in 3 mL eines Lösungsmittels für 24h bei 120°C gerührt. In einigen Fällen wurde dabei das Reaktionsgemisch durch den Zusatz von 500 mg Molsieben (3A) getrocknet. Die Ausbeuten wurden unter Verwendung von n-Tetradecan als internem Standard mit Hilfe von Gaschromatographie ermittelt.

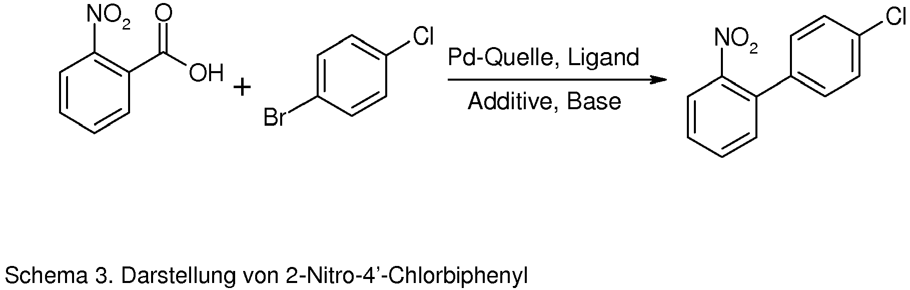

| Beispiel | Pd-Quelle | Ligand | Solvens | Base | Additive | Ausb. (%) |
|---|---|---|---|---|---|---|
| 1 | Pd(acac)₂ | PPh₃ | NMP | CuCO₃ | -- | 9 |
| 2 | PdCl₂ | PPh₃ | NMP | CuCO₃ | -- | 3 |
| 3 | Pd(OAc)₂ | PPh₃ | NMP | CuCO₃ | -- | 5 |
| 4 | Pd(acac)₂ | PPh₃ | NMP | CuCO₃ | KBr | 14 |
| 5 | Pd(acac)₂ | PPh₃ | NMP | CuCO₃ | NaF | 16 |
| 6 | Pd(acac)₂ | PPh₃ | NMP | CuCO₃ | LiF | 15 |
| 7 | Pd(acac)₂ | PPh₃ | NMP | CuCO₃ | KF | 32 |
| 8 | Pd(acac)₂ | PPh₃ | NMP | CuCO₃ | KF/3Å MS | 84 |
| 9 | Pd(acac)₂ | BINAP | NMP | CuCO₃ | KF/3Å MS | 76 |
| 10 | Pd(acac)₂ | DPPF | NMP | CuCO₃ | KF/3Å MS | 70 |
| 11 | Pd(acac)₂ | P(*p*-MeOPh)₃ | NMP | CuCO₃ | KF/3Å MS | 71 |
| 12 | Pd(acac)₂ | P(Cy)₃ | NMP | CuCO₃ | KF/3Å MS | 60 |
| 13 | Pd(acac)₂ | Bipyridine | NMP | CuCO₃ | KF/3Å MS | 35 |
| 14 | Pd(acac)₂ | P(*i*-propyl)Ph₂ | NMP | CuCO₃ | KF/3Å MS | 98 |
| 15 | Pd(acac)₂ | P(*i*-propyl)Ph₂ | NMP | Ag₂CO₃ | KF/3Å MS | 45 |
| 16 | Pd(acac)₂ | P(*i*-propyl)Ph₂ | DMSO | CuCO₃ | KF/3Å MS | 69 |
| 17 | Pd(acac)₂ | P(*i*-propyl)Ph₂ | DMPU | CuCO₃ | KF/3Å MS | 62 |
| 18 | Pd(acac)₂ | P(*i*-propyl)Ph₂ | Diglyme | CuCO₃ | KF/3Å MS | 40 |

### Beispiele 19-25

Aus den Beispielen 19-25, Tabelle 2 geht hervor, dass in einer zweiten Verfahrensvariante die . Umsetzung von *in situ* erzeugten Kaliumcarboxylaten mit Arylhalogeniden bereits mit äußerst geringen Mengen von Katalysatorssystemen bestehend aus Kupfer(I)salzen, Palladium(II)salzen und Aminliganden in hoher Effizienz erfolgt. Dabei wurde jeweils zunächst 1,5 mmol o-Nitrobenzoesäure mit einer stöchiometrischen Menge Kaliumcarbonat deprotoniert und das Reaktionswasser destillativ entfernt. Das resultierende Carbonsäuresalz wurde dann mit 1,0 mmol 4-Chlor-1-brombenzol in Gegenwart von 0,02 mmol Palladium(II)acetylacetonat, der angegebenen Menge Kupfer(I)halogenid und der angegebenen Menge des Aminliganden in NMP für 24h bei 160°C gerührt. Eventuell verbliebene Feuchtigkeit wurde dabei durch den Zusatz von 500 mg Molsieben (3A) gebunden. Die Ausbeuten wurden unter Verwendung von n-Tetradecan als internem Standard mit Hilfe von Gaschromatographie ermittelt.

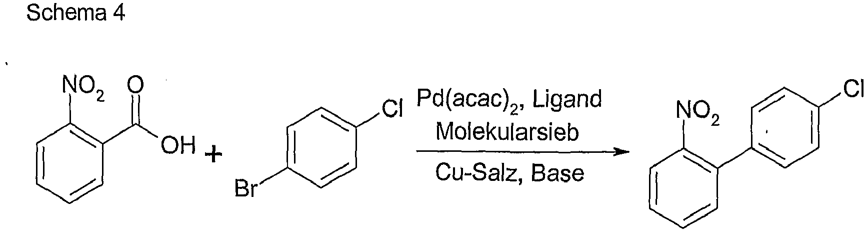

**Tabelle 2**

| Beispiel | Pd(acac)₂ (mmol) | Ligand (mmol) | Cu-Salz (mmol) | Ausb. (%) |
|---|---|---|---|---|
| 19 | 0,02 | Dipyridyl(0,1) | CuI (0,1) | 99 |
| 20 | 0,02 | Dipyridyl(0,1) | CuBr(0,1) | 95 |
| 21 | 0,02 | Dipyridyl(0,1) | CuCI(0,1) | 95 |
| 22 | 0,02 | 4,4'-Dimethyl-2,2'-dipyridyl(0,1) | CuI (0,1) | 98 |
| 23 | 0,02 | Phenanthrolin (0,1) | CuI (0,1) | 99 |
| 24 | 0,01 | Phenanthrolin (0,05) | CuI (0,03) | 97 |
| 25 | 0,005 | Phenanthrolin (0,05) | CuI (0,01) | 80 |

### Beispiele 26-55

Beispiele 26-55 aus Tabelle 3, die gemäß Schema 5 in einem Maßstab von 1 mmol durchgeführt wurden, demonstrieren die breite Anwendbarkeit des erfindungsgemäßen Verfahrens. Die Produkte wurden zur Bestimmung der Ausbeuten nach wässriger Aufarbeitung über Kieselgel chromatographiert und mit Hilfe von NMR, MS, HRMS eindeutig charakterisiert.

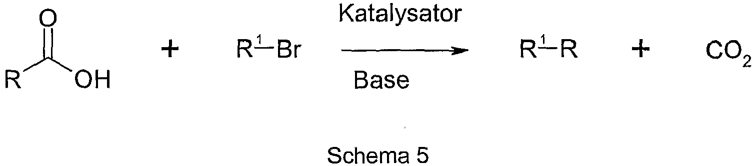

**Tabelle 3**

| | R | R¹ | Bedingungen | Ausb. (%) |
|---|---|---|---|---|
| 26 | 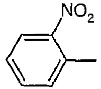 | 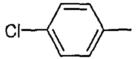 | Pd(acac)₂ (0.02mmol), CuCO₃ (1.5mmol), P(*iso*-propyl)Ph₂ (0.06mmol), KF (1.5mmol), Molsiebe (3A, 500mg), NMP (3ml), 120°, 24h | 98 |
| 27 | 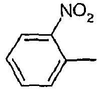 | 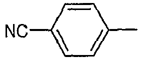 | Pd(acac)₂ (0.02mmol), CuCO₃ (1.5mmol), P(*iso*-propyl)Ph₂ (0.06mmol), KF (1.5mmol), Molsiebe (3A, 500mg), NMP (3ml), 120°, 24h | 96 |
| 28 | 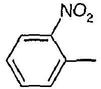 | 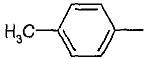 | Pd(acac)₂ (0.02mmol), CuCO₃ (1.5mmol), P(*iso*-propyl)Ph₂ (0.06mmol), KF (1.5mmol), Molsiebe (3A, 500mg), NMP (3ml), 120°, 24h | 97 |
| 29 | 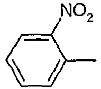 | 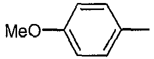 | Pd(acac)₂ (0.02mmol), CuCO₃ (1.5mmol), P(*iso*-propyl)Ph₂ (0.06mmol), KF (1.5mmol), Molsiebe (3A, 500mg), NMP (3ml), 120°, 24h | 79 |
| 30 | 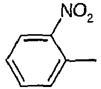 | 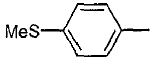 | siehe Beispiel 29 | 82 |
| 31 | 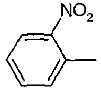 | 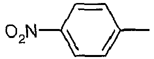 | siehe Beispiel 29 | 95 |
| 32 | 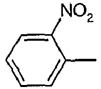 | 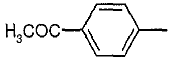 | siehe Beispiel 29 | 86 |
| 33 | 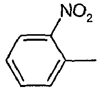 | 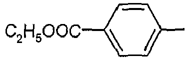 | siehe Beispiel 29 | 97 |
| 34 | 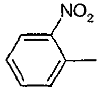 | 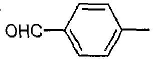 | siehe Beispiel 29 | 93 |
| 35 | 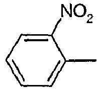 | 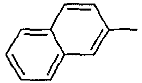 | siehe Beispiel 29 | 94 |
| 36 | 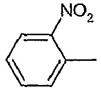 | 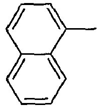 | siehe Beispiel 29 | 88 |
| 37 | 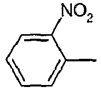 | 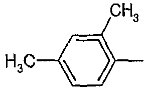 | siehe Beispiel 29 | 93 |
| 38 | 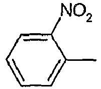 | 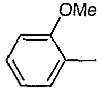 | siehe Beispiel 29 | 80 |
| 39 | 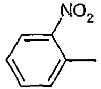 | 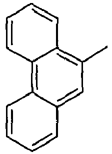 | siehe Beispiel 29 | 86 |
| 40 | 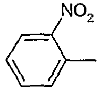 | 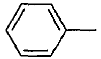 | siehe Beispiel 29 | 85 |
| 41 | 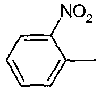 | 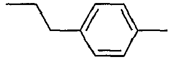 | siehe Beispiel 29 | 95 |
| 42 | 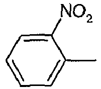 | 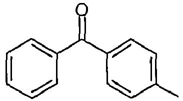 | siehe Beispiel 29 | 94 |
| 43 | 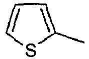 | 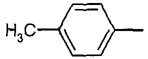 | Pd(acac)₂(0.02mmol), CuI (1.2mmol), dipyridyl (0.1mmol), K₂CO₃ (1.2mmol), Molsiebe (3A, 500mg), NMP (3ml), 160°, 24h | 82 |
| 44 | 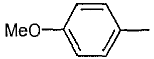 | 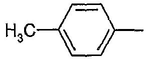 | Pd(acac)₂ (0.02mmol), CuCO₃ (1.5mmol), P(*iso*-propyl)Ph₂ (0.06mmol), KF (1.5mmol), NMP (3ml), 160°, 24h | 46 |
| 45 | 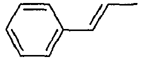 | 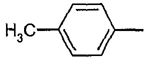 | siehe Beispiel 44 | 82 |
| 46 | 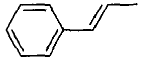 | 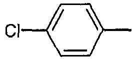 | siehe Beispiel 44 | 85 |
| 47 | 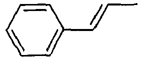 | 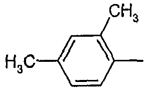 | siehe Beispiel 44 | 88 |
| 48 | 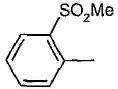 | 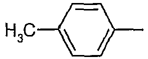 | siehe Beispiel 43 | 97 |
| 49 | 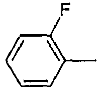 | 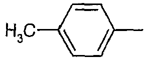 | siehe Beispiel 43 | 82 |
| 50 | 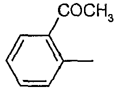 | 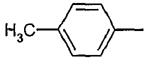 | siehe Beispiel 43 | 83 |
| 51 | 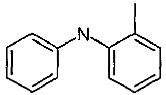 | 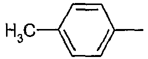 | siehe Beispiel 43 | 91 |
| 52 | 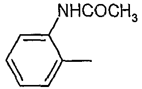 | 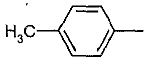 | siehe Beispiel 43 | 30 |
| 53 | 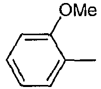 | 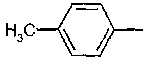 | siehe Beispiel 43 | 29 |
| 54 | 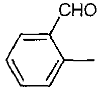 | 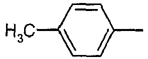 | siehe Beispiel 43 | 60 |
| 55 | 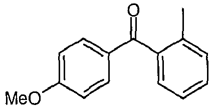 | 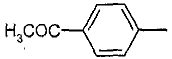 | siehe Beispiel 43 | 70 |

### Beispiele mit katalytischer Menge Kupfer und katalytischer Menge Palladium

Beispiele 56-71 aus Tabelle 4, die gemäß Schema 5 mit 1 mmol Arylbromid und 1.2 mmol Carbonsäure durchgeführt wurden, demonstrieren, dass beide Übergangsmetalle unabhängig von den Substraten lediglich in katalytischen Mengen benötigt werden. Die Produkte wurden zur Bestimmung der Ausbeuten nach wässriger Aufarbeitung über Kieselgel chromatographiert und mit Hilfe von NMR, MS, HRMS eindeutig charakterisiert.

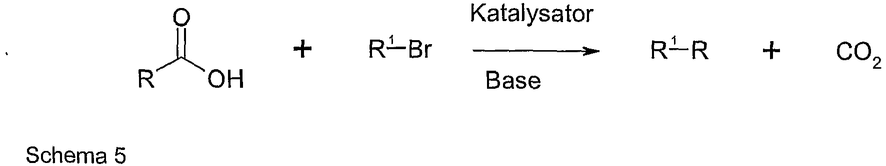

| | R | R¹ | Bedingungen | Ausb. (%) |
|---|---|---|---|---|
| 56 | 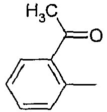 | 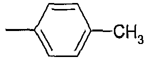 | Cs₂CO₃ (1.2 mmol), CuCl (0.15 mmol), Pd(acac)₂ (0.02 mmol), 1,10-Phenanthrolin (0.3 mmol), PPh₃ (0,04 mmol), Molsiebe (3Å, 250 mg) | 66 |
| 57 | 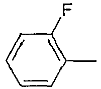 | 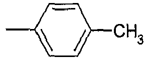 | Cs₂CO₃ (1.2 mmol), CuCl (0.10 mmol), Pd(acac)₂ (0.02 mmol), 1,10-Phenanthrolin (0.2 mmol), PPh₃ (0,06 mmol), Molsiebe (3Å, 250 mg) | 67 |
| 58 | 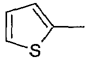 | 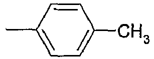 | K₂CO₃ (1.2 mmol), CuCl (0.10 mmol), Pd(acac)₂ (0.02 mmol), 1,10-Phenanthrolin (0.1 mmol), PPh₃ (0,06 mmol), Molsiebe (3Å, 250 mg) | 65 |
| 59 | 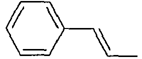 | 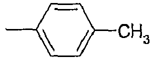 | Siehe Beispiel 56 | 91 |
| 60 | 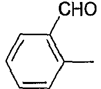 | 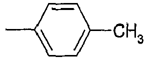 | Siehe Beispiel 56 | 28 |
| 61 | 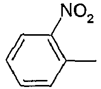 | 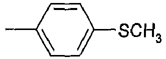 | Siehe Beispiel 24, mit 0.1 mmol CuI | 98 |
| 62 | 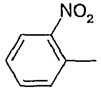 | 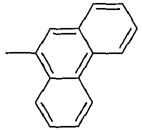 | Siehe Beispiel 24 | 99 |
| 63 | 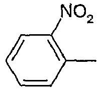 | 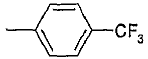 | Siehe Beispiel 24 | 93 |
| 64 | 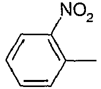 | 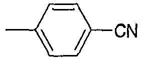 | Siehe Beispiel 24 | 93 |
| 65 | 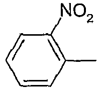 | 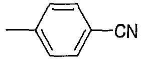 | Siehe Beispiel 24, aus dem Arylchlorid | 96 |
| 66 | 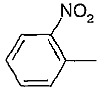 | 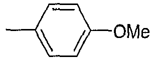 | Siehe Beispiel 24 | 68 |
| 67 | 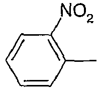 | 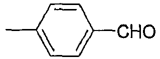 | Siehe Beispiel 24 | 78 |
| 68 | 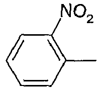 | 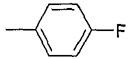 | Siehe Beispiel 24 | 97 |
| 69 | 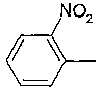 | 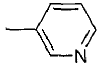 | Siehe Beispiel 24 | 98 |
| 70 | 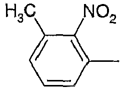 | 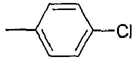 | Siehe Beispiel 24 | 74 |
| 71 | 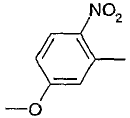 | 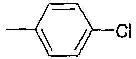 | Siehe Beispiel 24 | 77 |

## Patentansprüche

1. Verfahren zur decarboxylierenden C-C-Verknüpfung durch Umsetzung von Carbonsäuresalzen mit Kohlenstoffelektrophilen in Gegenwart von Übergangsmetallen oder -verbindungen als Katalysator, wobei der Katalysator als Katalysatorsystem zwei unterschiedliche Übergangsmetalle und/oder Übergangsmetallverbindungen enthält,
wobei ein Übergangsmetall Cu ist;
wobei das weitere Übergangsmetall Pd ist;
wobei mindestens eines der Metalle durch weitere Liganden aus der Reihe Amine, Phosphine, N-heterocyclische Carbene, Nitrile, Olefine stabilisiert ist; wobei das Carbonsäuresalz der allgemeinen Formel 1 entspricht, wobei n, m, q, p Zahlen zwischen 1 und 6 sind und r eine Zahl zwischen 0 und 6 ist, M wahlweise ein Metall oder eine organische Verbindung ist, R ein organischer Rest und Y⁻ ein Anion ist; 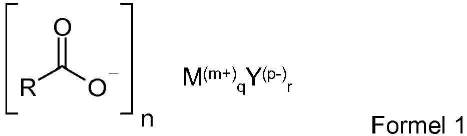 und wobei das Kohlenstoffelektrophil der allgemeinen Formel R¹-X entspricht, wobei R¹ ein organischer Rest ist und der Substituent X wählbar ist aus der Reihe: Halogenide aus der Reihe I, Br, Cl sowie Pseudohalogenide aus der Reihe Toluolsulfonat, Methylsulfonat, Trifluorsulfonat, Trifluoracetat, Carboxylat oder [N₂]⁺, oder wobei das Kohlenstoffelektrophil ein Carbonylderivat aus der Reihe Carbonsäurechlorid, Carbonsäureanhydrid, Aldehyd, Keton, Ester, α,β-ungesättigtes Aldehyd, α,β-ungesättigtes Keton, α,β-ungesättigter Ester ist;
wobei der Substituent R in der allgemeinen Formel 1 ausgewählt ist aus der Reihe lineare, verzweigte und zyklische C₁ - C₂₀-Alkyl, lineare, verzweigte und zyklische C₂ - C₂₀- Alkenyl- sowie C₆- C₂₀-Aryl und Heteroaryl aus der Reihe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, und seinerseits weitere Substituenten aus der Reihe lineare, verzweigte und zyklische C₁ - C₁₀-Alkyl, lineare, verzweigte und zyklische C₂ - C₂₀- Alkenyl- sowie C₆-C₁₀-Aryl und Heteroaryl, lineare, verzweigte und zyklische C₁₋C₁₀-Alkyloxy oder C₁₋C₁₀-Aryloxy, lineare, verzweigte und zyklische C₁ - C₁₀ Alkyl- oder C₁ - C₁₀-Arylaminocarbonyl, lineare, verzweigte und zyklische C₁ - C₁₀ Acyl, lineare, verzweigte und zyklische C₁ - C₁₀ Dialkylamino, C₁ - C₁₀ Arylamino, Formyl, Oxo, Thio, Hydroxy, Carboxyl, Nitro, Cyano, Nitroso, und Halogene wie F, Cl, Br und I tragen kann; und
wobei der Substituent R¹ des Kohlenstoffelektrophils R¹-X ausgewählt ist aus der Reihe lineare, verzweigte und zyklische C₁ - C₂₀-Alkyl, lineare, verzweigte und zyklische C₂ - C₂₀- Alkenyl- sowie C₆- C₂₀-Aryl und Heteroaryl aus der Reihe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, und seinerseits weitere Substituenten aus der Reihe lineare, verzweigte und zyklische C₁ - C₁₀-Alkyl, lineare, verzweigte und zyklische C₂ - C₂₀- Alkenyl- sowie C₆ - C₁₀-Aryl und Heteroaryl, lineare, verzweigte und zyklische C₁ - C₁₀-Alkyloxy oder C₁ - C₁₀-Aryloxy, lineare, verzweigte und zyklische C₁ - C₁₀ Alkyl- oder C₁ - C₁₀-Arylaminocarbonyl, lineare, verzweigte und zyklische C₁ - C₁₀ Acyl, lineare, verzweigte und zyklische C₁ - C₁₀ Dialkylamino, C₁ - C₁₀ Arylamino, Formyl, Oxo, Thio, Hydroxy, Carboxyl, Nitro, Cyano, Nitroso, und Halogene wie F, Cl, Br und I tragen kann.

2. Verfahren nach Anspruch 1, wobei die Übergangsmetallverbindung ein Kupfer(I)salz ist.

3. Verfahren nach Anspruch 1 oder 2 , wobei die Übergangsmetallverbindung Palladium(II)acetylacetonat ist.

4. Verfahren nach Anspruch 1, 2 oder 3, wobei ein cyclisches Amin als Ligand verwendet wird.

5. Verfahren nach Anspruch 4, wobei es sich bei dem Liganden um ein wahlweise substituiertes, chelatisierendes cyclisches Amin aus der Reihe Phenanthrolin, Bipyridin und Terpyridin handelt.

6. Verfahren nach einem der Ansprüche 1-5, wobei von beiden Katalysatoren unabhängig voneinander Mengen von 0,001 mol% bis 100 mol% bezogen auf das Kohlenstoffelektrophil eingesetzt werden.

7. Verfahren nach einem der Ansprüche 1-5, wobei von beiden Katalysatoren unabhängig voneinander Mengen von 0,01 mol% bis 5 mol% bezogen auf das Kohlenstoffelektrophil eingesetzt werden.

8. Verfahren nach einem der Ansprüche 1-7, wobei das Gegenion M^{(m+)} in der allgemeinen Formel 1 ein Kation eines Metalls aus der Reihe Li, Na, K, Mg, Ca, B, Al, Ag, Cu, Mn, Fe, Co, Ni, Mo, Ru ist.

9. Verfahren nach einem der Ansprüche 1-8, wobei der Substituent R in der allgemeinen Formel 1 wählbar ist aus der Reihe lineare, verzweigte und zyklische C₂ - C₂₀- Alkenyl- sowie C₆- C₂₀-Aryl und Heteroaryl aus der Reihe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, und seinerseits weitere Substituenten aus der Reihe lineare, verzweigte und zyklische C₁ - C₁₀-Alkyl, lineare, verzweigte und zyklische C₂ - C₂₀- Alkenyl- sowie C₆-C₁₀-Aryl und Heteroaryl, lineare, verzweigte und zyklische C₁-C₁₀-Alkyloxy oder C₁-C₁₀-Aryloxy, lineare, verzweigte und zyklische C₁ - C₁₀ Alkyl- oder C₁ - C₁₀-Arylaminocarbonyl, lineare, verzweigte und zyklische C₁ - C₁₀ Acyl, lineare, verzweigte und zyklische C₁ - C₁₀ Dialkylamino, C₁ - C₁₀ Arylamino, Formyl, Oxo, Thio, Hydroxy, Carboxyl, Nitro, Cyano, Nitroso, und Halogene wie F, Cl, Br und I tragen kann.

10. Verfahren nach einem der Ansprüche 1-9, wobei der Substituent R¹ des Kohlenstoffelektrophils R¹-X wählbar ist aus der Reihe lineare, verzweigte und zyklische C₂-C₂₀- Alkenyl- sowie C₆- C₂₀-Aryl und Heteroaryl aus der Reihe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, und seinerseits weitere Substituenten aus der Reihe lineare, verzweigte und zyklische C₁-C₁₀-Alkyl, lineare, verzweigte und zyklische C₂-C₂₀- Alkenyl- sowie C₆-C₁₀-Aryl und Heteroaryl, lineare, verzweigte und zyklische C₁-C₁₀-Alkyloxy oder C₁-C₁₀-Aryloxy, lineare, verzweigte und zyklische C₁-C₁₀ Alkyl- oder C₁ - C₁₀-Arylaminocarbonyl, lineare, verzweigte und zyklische C₁-C₁₀ Acyl, lineare, verzweigte und zyklische C₁-C₁₀ Dialkylamino, C₁-C₁₀ Arylamino, Formyl, Oxo, Thio, Hydroxy, Carboxyl, Nitro, Cyano, Nitroso, und Halogene wie F, Cl, Br und I tragen kann.

11. Verfahren nach einem der Ansprüche 1-10, wobei die Reaktionstemperatur 80°C bis 200°C beträgt.

12. Verfahren nach einem der Ansprüche 1-10, wobei die Reaktionstemperatur 120°C bis 160°C beträgt.

13. Verfahren nach einem der Ansprüche 1 bis 12, wobei Spuren von Wasser während der Reaktion entfernt werden.

14. Katalysatorsystem zur decarboxylierenden C-C-Verknüpfung von Carbonsäuresalzen mit Kohlenstoffelektrophilen, bestehend aus einer Palladiumverbindung und einer Kupferverbindung und einem wahlweise substituierten, chelatisierenden cyclischen Amin aus der Reihe Phenanthrolin, Bipyridin und Terpyridin.

## Claims

1. A process for decarboxylating C-C bond formation by reacting carboxylic acid salts with carbon electrophiles in the presence of transition metals or transition metal compounds as a catalyst wherein the catalyst as catalyst system comprises two different transition metals and/or transition metal compounds,
wherein one transition metal compound is Cu,
wherein the further transition metal compound is Pd,
wherein at least one of the metals is stabilized by further ligands from the group of amines, phosphines, N-heterocyclic carbenes, nitriles, olefins
wherein the carboxylic acid salt corresponds to the general Formula 1 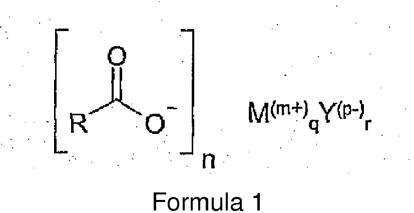 where n, m, q, p are numbers from 1 to 6 and r is a number from 0 to 6, M is either a metal or an organic compound, R is an organic radical and Y⁻ is an anion,
wherein the carbon electrophile corresponds to the general formula R¹-X where R¹ is an organic radical and the substituent X can be selected from the group of halides from the group of I, Br, Cl, and pseudohalides from the group of toluenesulfonate, methylsulfonate, trifluorosulfonate, trifluoroacetate, carboxylate or [N₂]⁺, or
wherein the carbon electrophile is a carbonyl derivative from the group of carbonyl chloride, carboxylic anhydride, aldehyde, ketone, ester, α,β-unsaturated aldehyde, α,β-unsaturated ketone, α,β-unsaturated ester
wherein the substituent R in the general formula 1 is selected from the group of linear, branched and cyclic C₁-C₂₀-alkyl, linear, branched and cyclic C₂-C₂₀-alkenyl, and also C₆-C₂₀-aryl and heteroaryl from the group of pyridine, pyrimidine, pyridazine, pyrazine, triazine, tetrazine, pyrrole, pyrazole, isoxazole, imidazole, oxazole, thiazole, thiophene, furan, and may in turn bear further substituents from the group of linear, branched and cyclic C₁-C₁₀-alkyl, linear, branched and cyclic C₂-C₂₀-alkenyl, and also C₆-C₁₀-aryl and heteroaryl, linear, branched and cyclic C₁₋C₁₀-alkyloxy or C₁-C₁₀-aryloxy, linear, branched and cyclic C₁₋C₁₀-alkyl- or C₁-C₁₀-arylaminocarbonyl, linear, branched and cyclic C₁₋C₁₀-acyl, linear, branched and cyclic C₁-C₁₀-dialkylamino, C₁₋C₁₀-arylamino, formyl, oxo, thio, hydroxyl, carboxyl, nitro, cyano, nitroso, and halogens such as F, Cl, Br and I,
wherein the substituent R¹ of the carbon electrophile is selected from the group of linear, branched and cyclic C₁-C₂₀-alkyl, linear, branched and cyclic C₂-C₂₀-alkenyl, and also C₆-C₂₀-aryl and heteroaryl from the group of pyridine, pyrimidine, pyridazine, pyrazine, triazine, tetrazine, pyrrole, pyrazole, isoxazole, imidazole, oxazole, thiazole, thiophene, furan, and may in turn bear further substituents from the group of linear, branched and cyclic C₁-C₁₀-alkyl, linear, branched and cyclic C₂-C₂₀-alkenyl, and also C₆-C₁₀-aryl and heteroaryl, linear, branched and cyclic C₁₋C₁₀-alkyloxy or C₁-C₁₀-aryloxy, linear, branched and cyclic C₁-C₁₀-alkyl- or C₁-C₁₀-arylaminocarbonyl, linear, branched and cyclic C₁-C₁₀-acyl, linear, branched and cyclic C₁-C₁₀-dialkylamino, C₁-C₁₀-arylamino, formyl, oxo, thio, hydroxyl, carboxyl, nitro, cyano, nitroso, and halogens such as F, Cl, Br and I.

2. The process as claimed in claim 1, wherein the transition metal compound is a copper (I) salt.

3. The process as claimed in claim 1 or 2, wherein the transition metal compound is palladium(II) acetylacetonate.

4. The process as claimed in claim 1, 2 or 3, wherein a cyclic amine is used as the ligand.

5. The process as claimed in claim 4, wherein the ligand is an optionally substituted chelating cyclic amine from the group of phenanthroline, bipyridine and terpyridine.

6. The process as claimed in anyone of claims 1 to 5, wherein mutually independent amounts of the two catalysts of from 0.001 mol% to 100 mol% based on the carbon electrophile are used.

7. The process as claimed in anyone of claims 1 to 5, wherein mutually independent amounts of the two catalysts of from 0.01 mol% to 5 mol% based on the carbon electrophile are used.

8. The process as claimed in anyone of claims 1 to 7, wherein the counterion M^{(m+)} is a cation of a metal from the group of Li, Na, K, Mg, Ca, B, Al, Ag, Cu, Mn, Fe, Co, Ni, Mo, Ru.

9. The process as claimed in anyone of claims 1 to 8, wherein the substituent R in the general formula 1 can be selected from the group of linear, branched and cyclic C₂-C₂₀-alkenyl, and also C₆-C₂₀-aryl and heteroaryl from the group of pyridine, pyrimidine, pyridazine, pyrazine, triazine, tetrazine, pyrrole, pyrazole, isoxazole, imidazole, oxazole, thiazole, thiophene, furan, and may in turn bear further substituents from the group of linear, branched and cyclic C₁-C₁₀-alkyl, linear, branched and cyclic C₂-C₂₀-alkenyl, and also C₆-C₁₀-aryl and heteroaryl, linear, branched and cyclic C₁-C₁₀-alkyloxy or C₁₋C₁₀-aryloxy, linear, branched and cyclic C₁₋C₁₀-alkyl- or C₁-C₁₀-arylaminocarbonyl, linear, branched and cyclic C₁-C₁₀-acyl, linear, branched and cyclic C₁-C₁₀-dialkylamino, C₁-C₁₀-arylamino, formyl, oxo, thio, hydroxyl, carboxyl, nitro, cyano, nitroso, and halogens such as F, Cl, Br and I.

10. The process as claimed in anyone of claims 1 to 9, wherein the substituent R¹ of the carbon electrophile can be selected from the group of linear, branched and cyclic C₂-C₂₀-alkenyl, and also C₆-C₂₀-aryl and heteroaryl from the group of pyridine, pyrimidine, pyridazine, pyrazine, triazine, tetrazine, pyrrole, pyrazole, isoxazole, imidazole, oxazole, thiazole, thiophene, furan, and may in turn bear further substituents from the group of linear, branched and cyclic C₁-C₁₀-alkyl, linear, branched and cyclic C₂-C₂₀-alkenyl, and also C₆-C₁₀-aryl and heteroaryl, linear, branched and cyclic C₁-C₁₀-alkyloxy or C₁-C₁₀-aryloxy, linear, branched and cyclic C₁-C₁₀-alkyl- or C₁-C₁₀-arylaminocarbonyl, linear, branched and cyclic C₁-C₁₀-acyl, linear, branched and cyclic C₁-C₁₀-dialkylamino, C₁-C₁₀-arylamino, formyl, oxo, thio, hydroxyl, carboxyl, nitro, cyano, nitroso, and halogens such as F, Cl, Br and I.

11. The process as claimed in anyone of claims 1 to 10, wherein the reaction temperature is from 80°C to 200°C.

12. The process as claimed in anyone of claims 1 to 10, wherein the reaction temperature is from 120°C to 160°C.

13. The process as claimed in anyone of claims 1 to 12, wherein traces of water are removed during the reaction.

14. Catalyst system for decarboxylating C-C bond formation of carboxylic acid salts with carbon electrophiles, consisting of a palladium compound and a copper compound and an optionally substituted chelating cyclic amine from the group of phenanthroline, bipyridine and terpyridine.

## Revendications

1. Procédé de liaison C-C décarboxylante par transformation de sels d'acide carboxylique avec des électrophiles de carbone en présence de métaux ou de composés de transition faisant office de catalyseur, le catalyseur en tant que système catalyseur contenant des métaux de transition et/ou des composés métalliques de transition différents ;
un métal de transition étant le Cu ;
l'autre métal de transition étant le Pd ;
dans lequel au moins un des métaux est stabilisé par d'autres ligands de la série des amines, phosphines, carbènes N-hétérocycliques, carbènes, nitriles, oléfines :
dans lequel le sel d'acide carboxylique répond à la formule générale 1, sachant que n, m, q, p sont des nombres compris entre 1 et 6 et que r est un nombre compris entre 0 et 6, M est au choix un métal ou un composé organique, R un radical organique et Y⁻ un anion ; 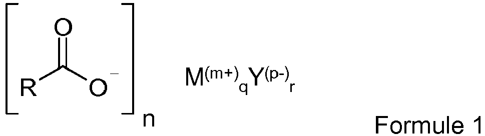 l'électrophile de carbone répondant à la formule générale R¹-X, R¹ dans un radical organique et le substituant X peut être sélectionné dans la série : halogénures de la série I, Br, Cl et pseudo-halogénures de la série toluène sulfonate, méthylsulfonate, trifluorsulfonate, trifluoracétate, carboxylate ou [N₂]⁺, ou l'électrophile de carbone étant un dérivé de carbonyle de la série : chlorure d'acide carboxylique, anhydride d'acide carboxylique, aldéhyde, cétone, ester, aldéhyde α,β-insaturé, cétone α,β-insaturée, ester α,β-insaturé ;
le substituant R étant sélectionné dans la formule générale 1 dans la série alkyle C₁ - C₂₀ ramifié et cyclique, alkényle C₂ - C₂₀ et aryle C₆- C₂₀ ramifié et cyclique et hétéroaryle de la série pyridine, pyrimidine, pyridazine, pyrazine, triazine, tétrazine, pyrrole, pyrazole, isoxazole, imidazole, oxazole, thiazole, thiophène, furane, et pouvant de son côté supporter d'autres substituants de la série de la série alkyle C₁ - C₁₀ linéaire, ramifié et cyclique, alkényle C₂- C₂₀ et aryle C₆-C₁₀ linéaire, ramifié et cyclique et hétéroaryle, alkyloxy C₁₋C₁₀ ou aryloxy C₁₋C₁₀ linéaire, ramifié et cyclique ou C₁₋C₁₀-aryloxy, alkyle C₁ - C₁₀ ou arylaminocarbonyle C₁ - C₁₀ linéaire, ramifié et cyclique, acyle C₁ - C₁₀ linéaire, ramifié et cyclique, dialkylamino C₁ - C₁₀ linéaire, ramifié et cyclique, formyle, oxo, thio, hydroxy, carboxyle, nitro, cyano, nitroso C₁ - C₁₀, et halogène, comme F, Cl, Br et I; et
le substituant R¹ de l'électrophile de carbone R¹-X étant sélectionné dans la série alkyle C₁ - C₂₀- linéaire, ramifié et cyclique, alkényle C₂ - C₂₀ linéaire, ramifié et cyclique et aryle C₆- C₂₀ et hétéroaryle de la série pyridine, pyrimidine, pyridazine, pyrazine, triazine, tétrazine, pyrrole, pyrazole, isoxazole, imidazole, oxazole, thiazole, thiophène, furane, et pouvant de son côté supporter d'autres substituants de la série alkyle C₁ - C₁₀- linéaire, ramifié et cyclique, alkényle C₂ - C₂₀ et aryle C₆ - C₁₀ linéaire, ramifié et cyclique et hétéroaryle, alkyloxy C₁ - C₁₀ linéaire ou aryloxy C₁ - C₁₀- linéaire, ramifié et cyclique ou alkyle C₁ - C₁₀ ou arylaminocarbonyle C₁ - C₁₀ linéaire, ramifié et cyclique, acyle C₁ - C₁₀ linéaire, ramifié et cyclique, dialkylamino C₁ - C₁₀ linéaire, ramifié et cyclique, arylamino, formyle, oxo, thio, hydroxy, carboxyle, nitro, cyano, nitroso C₁ - C₁₀, et halogène comme F, Cl, Br et I.

2. Procédé selon la revendication 1, dans lequel le composé métallique de transition est un sel de cuivre (I).

3. Procédé selon la revendication 1 ou 2, dans lequel le composé métallique de transition est du palladium(II)acétylacétonate.

4. Procédé selon la revendication 1, 2 ou 3, dans lequel une amine cyclique est utilisée comme ligand.

5. Procédé selon la revendication 4, dans lequel le ligand est une amine cyclique substituée chelatante au choix de la série phénanthroline, bipyridine et terpyridine.

6. Procédé selon une des revendications 1 à 5, dans lequel, des deux catalyseurs, on utilise indépendamment l'un de l'autre des quantités de 0,001 % en moles à 100 % en moles par rapport à l'électrophile de carbone.

7. Procédé selon une des revendications 1 à 5, dans lequel, des deux catalyseurs, on utilise indépendamment l'un de l'autre des quantités de 0,01 % en moles à 5 % en moles par rapport à l'électrophile de carbone.

8. Procédé selon une des revendications 1 à 7, dans lequel le contre-ion M^{(m+)} de formule générale 1 est un cation d'un métal de la série Li, Na, K, Mg, Ca, B, Al, Ag, Cu, Mn, Fe, Co, Ni, Mo, Ru.

9. Procédé selon une des revendications 1 à 8, dans lequel le substituant R peut être sélectionné dans la formule générale 1 dans la série alkényle C₂ - C₂₀ et aryle C₆-C₂₀ linéaire, ramifié et cyclique et hétéroaryle de la série pyridine, pyrimidine, pyridazine, pyrazine, triazine, tétrazine, pyrrole, pyrazole, isoxazole, imidazole, oxazole, thiazole, thiophène, furane, et peut de son côté supporter d'autres substituants de la série alkyle C₁ - C₁₀ linéaire, ramifié et cyclique, alkényle C₂ - C20 et aryle C6-C₁₀ linéaire, ramifié et cyclique et hétéroaryle, alkyloxy C₁-C₁₀ ou aryloxy C₁-C₁₀ linéaire, ramifié et cyclique, alkyle C₁ - C₁₀ ou arylaminocarbonyle C₁ - C₁₀ linéaire, ramifié et cyclique, acyle C₁ - C₁₀ linéaire, ramifié et cyclique, dialkylamino C₁ - C₁₀ linéaire, ramifié et cyclique, arylamino, formyle, oxo, thio, hydroxy, carboxyle, nitro, cyano, nitroso C₁ - C₁₀, et halogène comme F, Cl, Br et I.

10. Procédé selon une des revendications 1 à 9, dans lequel le substituant R¹ de l'électrophile de carbone R¹-X peut être sélectionné dans la série alkényle C₂-C₂₀ et aryle C₆- C₂₀ linéaire, ramifié et cyclique et hétéroaryle de la série pyridine, pyrimidine, pyridazine, pyrazine, triazine, tétrazine, pyrrole, pyrazole, isoxazole, imidazole, oxazole, thiazole, thiophène, furane, et peut de son côté supporter d'autres substituants de la série alkyle C₁- C₁₀ linéaire, ramifié et cyclique , alkényle C₂- C₂₀ et aryle C₆-C₁₀ linéaire, ramifié et cyclique et hétéroaryle, alkyloxy C₁-C₁₀ ou aryloxy C₁-C₁₀ linéaire, ramifié et cyclique, alkyle C₁-C₁₀ ou arylaminocarbonyle C₁ - C₁₀ linéaire, ramifié et cyclique, acyle C₁-C₁₀ linéaire, ramifié et cyclique , dialkylamino C₁-C₁₀, arylamino, formyle, oxo, thio, hydroxy, carboxyle, nitro, cyano, nitro C₁-C₁₀, et halogène comme F, Cl, Br et I.

11. Procédé selon une des revendications 1-10, dans lequel la température de réaction est de 80 °C à 200 °C.

12. Procédé selon une des revendications 1-10, dans lequel la température de réaction est de 120 °C à 160 °C.

13. Procédé selon une des revendications 1 à 12, dans lequel des traces d'eau sont éliminées pendant la réaction.

14. Système catalyseur pour la liaison C-C décarboxylante C-C de sels d'acide carboxylique avec des électrophile de carbone, composé d'un composé de palladium et d'une liaison de cuivre et d'une amine cyclique substituée chelatante au choix de la série phénanthroline, bipyridine et terpyridine.
